Europäisches Patentamt
European Patent Office
Office européen des brevets

(19)

(11) Publication number: **0 199 257 A2**

# EUROPEAN PATENT APPLICATION

(12)

(21) Application number: **86105165.4**

(51) Int. Cl.[4]: **C07K 5/06** , A23L 1/236

(22) Date of filing: **15.04.86**

(30) Priority: **15.04.85 US 723603**

(43) Date of publication of application: **29.10.86 Bulletin 86/44**

(84) Designated Contracting States: **AT BE CH DE FR GB IT LI NL SE**

(71) Applicant: **GENERAL FOODS CORPORATION**
**250 North Street**
**White Plains, N.Y. 10625(US)**

(72) Inventor: **Zanno, Paul Robert**
**2 April Lane**
**Nanuet New York(US)**
Inventor: **Barnett, Ronald Edward**
**73 E. Maple Avenue**
**Suffern New York(US)**
Inventor: **Roy, Glenn Michael**
**16 Trimble Street**
**Garnerville New York(US)**

(74) Representative: **Lehn, Werner, Dipl.-Ing. et al**
**Hoffmann, Eitle & Partner Patentanwälte**
**Arabellastrasse 4 (Sternhaus)**
**D-8000 München 81(DE)**

(54) **L-Aminodicarboxylic acid esters and an edible composition containing same as sweeteners.**

(57) Sweeteners of the Formula:

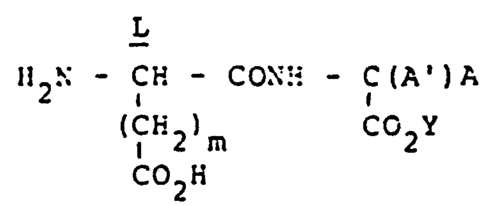

and food-acceptable salts thereof, where the substituents have the meaning disclosed in the specification and an edible composition containing same.

EP 0 199 257 A2

# L-AMINODICARBOXYLIC ACID ESTERS AND AN EDIBLE COMPOSITION CONTAINING SAME AS SWEETENERS

This invention relates to a novel group of compounds and more particularly to a novel group of compounds particularly well suited as sweeteners in edible foodstuff.

Sweetness is one of the primary taste cravings of both animals and humans. Thus, the utilization of sweetening agents in foods in order to satisfy this sensory desire is well established.

Naturally occuring carbohydrate sweeteners such as sucrose, are still the most widely used sweetening agents. While thse naturally occurring carbohydrates, i.e., sugars, generally fulfill the requirements of sweet taste, the abundant usage thereof does not occur without deleterious consequence, e.g., high caloric intake and nutritional imbalance. In fact, oftentimes the level of these sweeteners required in foodstuffs is far greater than the level of the sweetener that is desired for economic, dietetic or other functional consideration.

In an attempt to eliminate the disadvantages concomitant with natural sweeteners, considerable research and expense have been devoted to the production of artificial sweeteners, such as for example, saccharin, cyclamate, dihydrochalcone, aspartame, etc. While some of these artificial sweeteners satisfy the requirements of sweet taste without caloric input, and have met with considerable commercial success, they are not, however, without their own inherent disadvantages. For example, many of these artificial sweeteners have the disadvantages of high cost, as well as delay in the perception of the sweet taste, persistent lingering of the sweet taste, and very objectionable bitter, metallic aftertaste when used in food products.

Since it is believed that many disadvantages of artificial sweeteners, particularly aftertaste, is a function of the concentration of the sweetener, it has been previously suggested that these effects could be reduced or eliminated by combining artificial sweeteners such as saccharin, with other ingredients such as aspartame or natural sugars, such as sorbitol, dextrose, maltose, etc. These combined products, however, have not been entirely satisfactory either. Some U.S. Patents which disclose sweetener mixtures include for example, U.S. Patent No. 4,228,198; U.S. Patent No. 4,158,068; U.S. Patent No. 4,154,862; and U.S. Patent No. 3,717,477.

Accordingly, much work has continued in an attempt to develop and identify compounds that have a sweet taste and which will satisfy the need for better lower calorie sweeteners. Search continues for sweeteners that have intense sweetness, that is, deliver a sweet taste at low use levels and which will also produce enough sweetness at low levels to act as sole sweetener for most sweetener applications. Furthermore, the sweeteners sought must have good temporal and sensory qualities. Sweeteners with good temporal qualities produce a time-intensity sweetness response similar to natural sweeteners without lingering. Sweeteners with good sensory qualities lack undesirable off tastes and aftertaste. Furthermore, these compounds must be economical and safe to use.

In U.S. Patent No. 3,798,204, L-aspartyl-O-t-butyl-L-serine methyl ester and L-aspartyl-O-t-amyl-L-serine methyl ester are described as sweet compounds having significant sweetness.

In U.S. Patent No. 4,448,716 metal complex salts of dipeptide sweetners are disclosed. In the background of this patent a generic formula is described as an attempt to represent dipeptide sweeteners disclosed in five prior patents: U.S. Patent No. 3,475,403; U.S. Patent No. 3,492,131; Republic of South Africa Patent No. 695,083 published July 10, 1969; Republic of South Africa Patent No. 695,910 published August 14, 1969; and German Patent No. 2,054,554. The general formula attempting to represent these patents is as follows:

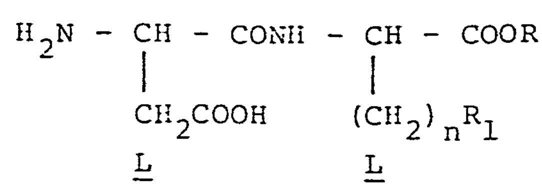

wherein R represents the lower alkyls, lower alkylaryls and cycloalkyls, n stands for integers 0 through 5, $R_1$ represents (a) phenyl group, (b) lower alkyls, (c) cycloalkyls, (d) $R_2$.

Where $R_2$ is hydroxy, lower alkoxy, lower alkyl, halogen, (e) $(S(O)_m$ (lower alkyl) where m is 0, 1 or 2 and provided n is 1 or 2, (f) $R_3$.

Where $R_3$ represents an hydroxy or alkoxy and (g) single or double unsaturated cycloalkyls with up to eight carbons. These compounds also are not entirely satisfactory in producing a high quality sweetness or in producing a sweet response at lower levels of sweetener.

Dipeptides of aspartyl-cysteine and aspartyl-methionine methyl esters are disclosed by Brussel, Peer and Van der Heijden in Chemical Senses and Flavour, 4, 141-152 (1979) and in Z. Lebensm. Untersuch-Forsch., 159, 337-343 (1975). The authors disclose the following dipeptides:

α-L-Asp-L-Cys(Me)-OMe

α-L-Asp-L-Cys(Et)-OMe

α-L-Asp-L-Cys(Pr)-OMe

α-L-Asp-L-Cys(i-Pr)-OMe

α-L-Asp-L-Cys(t-But)-OMe

α-L-Asp-L-Met-OMe

In U.S. Patent No. 4,399,163 to Brennan et al., sweeteners having the following formulas are disclosed:

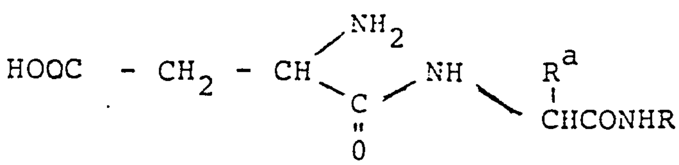

and physiologically acceptable cationic and acid addition salts thereof wherein

$R^a$ is $CH_2OH$ or $CH_2OCH_3$;

R is a branched member selected from the group consisting of fenchyl, diisopropylcarbinyl, d-methyl-t-butylcarbinyl, d-ethyl-t-butyl-carbinyl, 2-methylthio-2,4-dimethylpentan-3-yl, di-t-butyl-carbinyl,

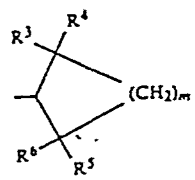
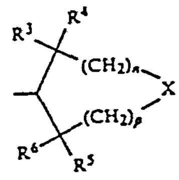

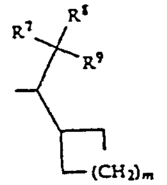
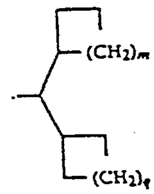

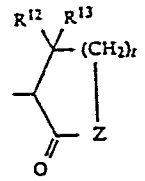

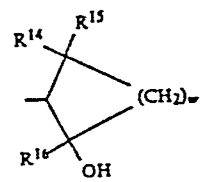

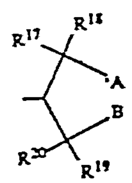

In a related patent, Patent No. 4,411,925, Brennan, et al. disclose compounds of the above general formula with R being defined hereinabove, except $R^a$ is defined as methyl, ethyl, n-propyl or isopropyl.

U.S. Patent No. 4,375,430 to Sklavounos discloses dipeptide sweeteners which are aromatic sulfonic acid salts of L-aspartyl-D-alaninoamides or L-aspartyl-D-serinamides.

European Patent Application No. 95772 to Tsau describe aspartyl dipeptide sweeteners of the formula:

$$\begin{array}{l} H_2N - \underset{\textstyle HO_2C - CH_2}{CH}CONH\underset{\textstyle R_2}{CH} - CO_2R' \end{array}$$

wherein R' is alkyl of 1 to 6 carbons, and $R_2$ is phenyl, phenylakylenyl or cyclohexylalkenyl, wherein the alkenyl group has 1 to 5 carbons.

Closely related is Patent No. 4,439,460 to Tsau, et al. which describes dipeptide sweeteners of the formula:

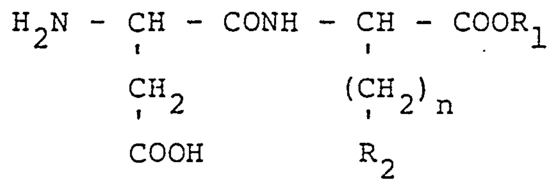

wherein n is an integer from 0 to 5, and $R_1$ is an alkyl, alkylaryl or alicyclic radical. Similar such compounds are described in many related patents, the major difference being the definition of $R_2$.

In U.S. Patent No. 3,978,034 to Sheehan, et al., $R_2$ is defined as cycloalkenyl or phenyl. U.S. Patent No. 3,695,898 to Hill defines $R_2$ as a mono-or a di-unsaturated alicyclic radical. Haas, et al. in Patent No. 4,029,701 define $R_2$ as phenyl, lower alkyl or substituted or unsubstituted cycloalkyl, cycloalkenyl or cycloalkdienyl, or $S(O)_m$ lower alkyl provided that n is 1 or 2 and m is 0 or 2. Closely related are U.S. Patent Nos. 4,448,716, 4,153,737, 4,031,258, 3,962,468, 3,714,139, 3,642,491, and 3,795,746.

U.S. Patent No. 3,803,223 to Mazur, et al. describe dipeptide sweeteners and anti-inflammatory agents having the formula:

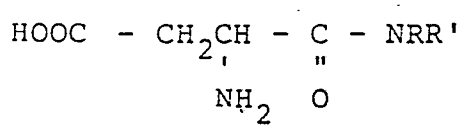

wherein R is hydrogen or a methyl radical and R' is a radical selected from the group consisting of alkyl, or

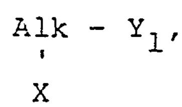

wherein Alk is a lower alkylene radical, X is hydrogen or hydroxy, and Y is a radical selected from the group consisting of cyclohexyl, naphthyl, furyl, pyridyl, indolyl, phenyl and phenoxy.

Goldkamp, et al. in U.S. Patent No. 4,011,260 describe sweeteners of the formula:

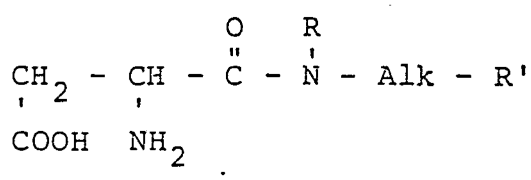

wherein R is hydrogen or a lower alkyl radical, Alk is a lower alkylene radical and R' is a carbocyclic radical. Closely related is U.S. Patent No. 3,442,431.

U.S. Patent No. 4,423,029 to Rizzi describes sweeteners of the formula:

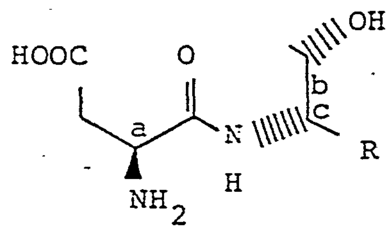

wherein R is $C_4$-$C_9$ straight, branched or cyclic alkyl, and wherein carbons a, b and c have the (S) configuration.

European Patent Application 48,051 describes dipeptide sweeteners of the formula:

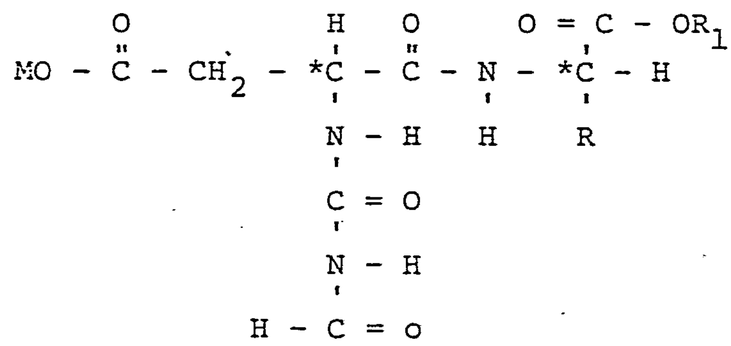

wherein M represents hydrogen, ammonium, alkali or alkaline earth,

R represents

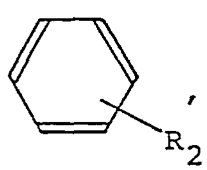 , 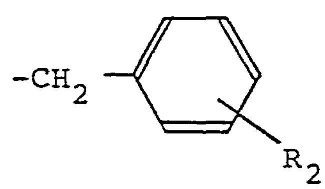 , 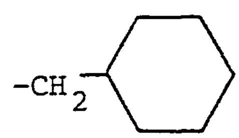

$R_1$ represents methyl, ethyl, propyl,

$R_2$ represents -OH, or $OCH_3$,

* signifies an L-optical configuration for this atom.

German Patent Application No. 7259426 discloses L-aspartyl-3-fenchylalanine methyl ester as a sweetening agent.

U.S. Patent No. 3,971,822 to Chibata, et al., disclose sweeteners having the formula:

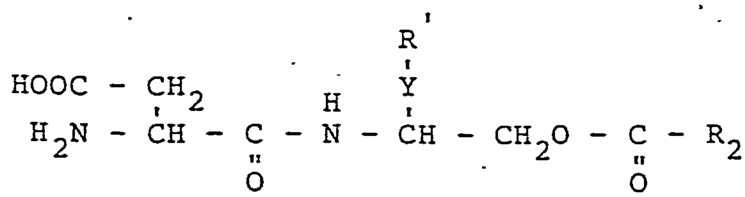

wherein R' is hydrogen or hydroxy, $R_2$ is alkyl of one to five carbon atoms, alkenyl of two to three carbon atoms, cycloalkyl of three to five carbon atoms or methyl cycloalkyl of four to six carbon atoms and Y is alkylene of one to four carbon atoms.

U.S. Patent No. 3,907,366 to Fujino, et al. discloses L-aspartyl-aminomalonic acid alkyl fenchyl diester and its' physiologically acceptable salts as useful sweeteners. Patent No. 3,959,245 disclose the 2-methyl cyclohexyl analog of the abovementioned patent.

U.S. Patent No. 3,920,626 discloses N-α L-aspartyl derivatives of lower alkyl esters of O-lower-alkanoyl-L-serine, β-alanine, γ-aminobutyric acid and D-β-aminobutyric acid as sweeteners.

Miyoshi, et al. in Bulletin of Chemical Society of Japan, 51, p. 1433-1440 (1978) disclose compounds of the following formula as sweeteners:

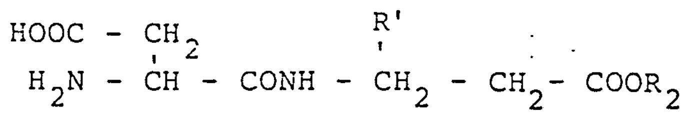

wherein R' is H, $CH_3$, $CO_2CH_3$, or benzyl and $R_2$ is lower alkyl or unsubstituted or substituted cycloalkyl.

European Patent Application 128,654 describes gem-diaminoalkane sweeteners of the formula:

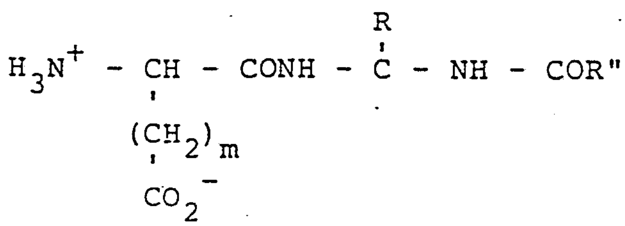

wherein m is 0 or 1, R is lower alkyl (substituted or unsubstituted), R' is H or lower alkyl, and R" is a branched alkyl, alkylcycloalkyl, cycloalkyl, polycycloalkyl, phenyl, or alkyl-substituted phenyl, and physically acceptable salts thereof.

U.S. Patent No. 3,801,563 to Nakajima, et al. disclose sweeteners of the formula:

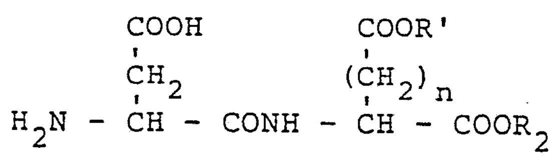

wherein R' is a branched or cyclic alkyl group of 3 to 8 carbon atoms, $R_2$ is a lower alkyl group of 1 to 2 carbon atoms and n is a integer of 0 or 1.

European Patent Application 34,876 describes amides of L-aspartyl-D-amino acid dipeptides of the formula:

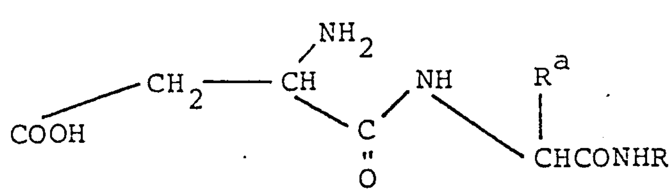

wherein $R^a$ is methyl, ethyl, n-propyl or isopropyl and R is a branched aliphatic, alicyclic or heterocyclic member which is branched at the alpha carbon atom and also branched again at one or both of the beta carbon atoms. These compounds are indicated to be of significant sweetness.

In the Journal of Medicinal Chemistry, 1984, Vol. 27, No. 12, pp. 1663-8, are described various sweetener dipeptide esters, including L-aspartyl-α-aminocycloalkane methyl esters.

The various dipeptide esters of the prior art have been characterized as lacking significant stability at low pH values and/or thermal stability. These characterstics have limited the scope of use of these sweeteners in food products which are of low pH values or are prepared or served at elevated temperatures.

Accordingly, it is desired to find compounds that provide quality sweetness when added to foodstuffs or pharmaceuticals at low levels and thus eliminate or greatly diminish the aforesaid disadvantages associated with prior art sweeteners.

SUMMARY OF THE INVENTION

The present new compounds are esters of certain α-aminodicarboxylic acids and α-aminoesters which are low calorie sweeteners that possess a high order of sweetness with pleasing taste and higher stability at acid pH and elevated temperatures compared to known dipeptide sweeteners.

This invention provides new sweetening compounds represented by the formula:

```
            L
            -
  H2N  -  CH  -  CONH  -  C(A')A
           |               |
        (CH2)m            CO2Y
           |
          CO2H
```

and food-acceptable salts thereof, wherein

A is hydrogen, alkyl containing 1-3 carbon atoms, hydroxyalkyl containing 1-3 carbon atoms or alkoxymethyl wherein the alkoxy contains 1-3 carbon atoms;

A' is hydrogen or alkyl containing 1-3 carbon atoms; alternatively

A and A' taken together with the carbon atom to which they are attached form cycloalkyl containing 3-4 carbon atoms;

Y is $-(CHR_2)_n-R_1$ or $-CHR_3R_4$;

$R_1$ is an alkyl-substituted cycloalkyl, cycloalkenyl bicycloalkyl or bicycloalkenyl wherein at least one alkyl is in the β-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

DESCRIPTION OF THE PREFERRED EMBODIMENTS

In accordance with the present invention, the preferred compounds are those in which $R_1$ is an alkyl-substituted cycloalkyl or bicycloalkyl containing 5 -7 ring carbon atoms and up to a total of 10 carbon atoms. Especially preferred are cycloalkyl substituted with at least one methyl group on the β and/or β' carbon atoms of the cycloalkyl ring. Particularly preferred cycloalkyls include cyclopropyl, cyclopentyl, and cyclohexyl and the preferred bicycloalkyl is fenchyl.

Also preferred are those compounds in which n = 0. In those compounds in which n = 1, $R_1$ is preferably a cyclopropyl group and $R_2$ is preferably tertiary butyl, isopropyl or cyclopropyl.

The groups representative of Y in the present new compounds include such groups as alkyl-substituted cycloalkyls, e.g., 1,2-dimethylcyclohexyl, 1,2-dimethylcyclopentyl, 1,2-dimethylcycloheptyl, 2,3-dimethylcyclopentyl, 2,3-dimethylcyclohexyl, 2,3-dimethylcycloheptyl, 2,4-dimethylcyclopentyl, 2,4-dimethylcyclohexyl, 2,4-dimethylcycloheptyl, 2,5-dimethylcyclopentyl, 2,5-dimethylcyclohexyl, 2,5-dimethylcycloheptyl, 2,6-dimethylcyclohexyl, 2,6-dimethylcycloheptyl, 2,7-dimethylcycloheptyl, 3,5-dimethylcyclopentyl, 4,5-dimethylcyclopentyl, 4,5-dimethylcycloheptyl, 3,6-dimethylcyclohexyl, 3,7-dimethylcycloheptyl, 4,6-dimethylcyclohexyl, 4,7-dimethylcycloheptyl, 5,6-dimethylcyclohexyl,

5,6-dimethylcyclohexyl, 5,7-dimethylcycloheptyl, 6,7-dimethylcycloheptyl, 2,2-dimethylcyclopentyl, 2,2-dimethylcyclohexyl, 2,2-dimethylcycloheptyl, 2,2,3-trimethylcyclopentyl, 2,2,3-trimethylcyclohexyl, 2,2,3-trimethylcycloheptyl, 2,2,4-trimethylcyclopentyl, 2,2,4-trimethylcyclohexyl, 2,2,4-trimethylcycloheptyl, 2,2,5-trimethylcyclopentyl, 2,2,5-trimethylcyclohexyl, 2,2,5-trimethylcycloheptyl, 2,3,3-trimethylcyclopentyl, 2,3,3-trimethylcyclohexyl,

2,3,3-trimethylcycloheptyl, 2,4,4-trimethylcyclopentyl,

2,4,4-trimethylcyclohexyl, 2,4,4-trimethylcycloheptyl,

1,2,3-trimethylcyclopentyl, 1,2,3-trimethylcyclohexyl,

1,2,3-trimethylcycloheptyl, 1,2,4-trimethylcyclopentyl,

1,2,4-trimethylcyclohexyl, 1,2,4-trimethylcycloheptyl,

1,2,5-trimethylcyclopentyl, 1,2,5-trimethylcyclohexyl,

1,2,5-trimethylcycloheptyl, 1,2,6-trimethylcyclohexyl,

1,2,6-trimethylcycloheptyl, 1,2,7-trimethylcycloheptyl,

2,3,4-trimethylcyclopentyl, 2,3,4-trimethylcyclohexyl,

2,3,4-trimethylcycloheptyl, 2,3,5-trimethylcyclopentyl,

2,3,5-trimethylcyclohexyl, 2,3,5-trimethylcycloheptyl,

2,3,6-trimethylcyclohexyl, 2,3,6-trimethylcycloheptyl,

2,3,7-trimethylcycloheptyl, 2,2,5,5-tetramethylcyclopentyl, 2,2,5,5-tetramethylcyclohexyl, 2,2,5,5-tetramethylcycloheptyl, 2,2,6,6-tetramethylcyclohexyl, 2,2,6,6-tetramethylcycloheptyl, 2,2,7,7-tetramethylcycloheptyl, 2,2,4,4-tetramethylcyclopentyl, 2,2,4,4-tetramethylcyclohexyl, 2,2,4,4-tetramethylcycloheptyl, 2,2,3,3-tetramethylcyclopentyl, 2,2,3,3-tetramethylcyclohexyl, 2,2,3,3-tetramethylcycloheptyl, 1,2,3,4-tetramethylcyclopentyl,

1,2,3,4-tetramethylcyclohexyl, 1,2,3,4-tetramethylcycloheptyl,

1,2,3,5-tetramethylcyclopentyl, 1,2,3,5-tetramethylcyclohexyl,

1,2,3,5-tetramethylcycloheptyl, 1,2,3,6-tetramethylcyclohexyl, 1,2,3,6-tetramethylcycloheptyl, 2,3,4,5-tetramethylcyclopentyl, 2,3,4,5-tetramethylcyclohexyl, 2,3,4,5-tetramethylcycloheptyl, 2,3,4,6-tetramethylcycloheptyl, 2,3,4,6-tetramethylcyclohexyl, 2,3,4,7-tetramethylcycloheptyl, 2,2,3,4-tetramethylcyclopentyl, 2,2,3,4-tetramethylcyclohexyl, 2,2,3,4-tetramethylcycloheptyl, 2,2,3,5-tetramethylcyclopentyl, 2,2,3,5-tetramethylcyclohexyl, 2,2,3,5-tetramethylcycloheptyl, 2,2,3,6-tetramethylcyclohexyl, 2,2,3,6-tetramethylcycloheptyl, 2,2,3,7-tetramethylcycloheptyl, 2,3,3,4-tetramethylcyclohexyl, 2,3,3,4-tetramethylcyclopentyl, 2,3,3,4-tetramethylcycloheptyl, 2,3,3,5-tetramethylcyclopentyl, 2,2,3,5-tetramethylcyclohexyl, 2,3,3,5-tetramethylcycloheptyl, 2,3,3,6-tetramethylcyclohexyl, 2,3,3,6-tetramethylcycloheptyl, 2,3,3,7-tetramethylcycloheptyl, 2,2,3,4-tetramethylcyclopentyl, 2,2,3,4-tetramethylcyclohexyl, 2,3,3,4-tetramethylcycloheptyl, 2,2,3,5-tetramethylcyclopentyl, 2,2,3,5-tetramethylcyclohexyl, 2,2,3,6-tetramethylcyclohexyl, 2,2,3,6-tetramethylcycloheptyl, 2,2,3,7-tetramethylcycloheptyl, 2,2,4,5-tetramethylcyclopentyl, 2,2,4,5-tetramethylcyclohexyl, 2,2,4,5-tetramethylcycloheptyl, 2,2,4,6-tetramethylcyclohexyl, 2,2,4,6-tetramethylcycloheptyl, 2,2,4,7-tetramethylcycloheptyl, dicyclopropylmethyl, t-butylcyclopropylmethyl, dicyclobutylmethyl, t-butylcyclobutylmethyl, etc.; β-alkyl-substituted cycloalkenes, e.g., 2-methyl-3-cyclohexenyl, 2-methyl-3-cyclopentenyl, 2-methyl-3-cycloheptenyl,2-methyl-4-cycloheptenyl, 5-methyl-3-cyclopentenyl, 2-methyl-2-cyclopentenyl, 2-methyl-2-cyclohexenyl, 2-methyl-2-cycloheptenyl, 2-methyl-2-cyclopentenyl, 6-methyl-2-cyclohexenyl, 7-methyl-2-cycloheptenyl, 2,3-dimethyl-2-cyclopentenyl, 2,3-dimethyl-2-cyclohexenyl, 2,4-dimethyl-2-cyclopentenyl, 2,4-dimethyl-2-cyclohexenyl, 2,5-dimethyl-2-cyclohexenyl, 2,5-dimethyl-2-cycloheptenyl, 2,6-dimethyl-2-cyclohexenyl, 2,6-dimethyl-3-cyclohexenyl, 2,5-dimethyl-3-cyclohexenyl, 2,5-dimethyl-2-cyclopentenyl, 2,4-dimethyl-3-cyclopentenyl, 2,4-dimethyl-3-cyclohexenyl, 4,5-dimethylcyclo-3-pentenyl, 5,5-dimethyl-3-cyclopentenyl, 6,6-dimethyl-3-cyclohexenyl, 1,2-dimethyl-3-cyclopentenyl, 1,2-dimethyl-3-cyclohexenyl, 1,5-dimethyl-3-cyclopentenyl, 2,2,6-trimethyl-3-cyclohexenyl, 2,2,5-trimethyl-3-cyclohexenyl, 2,5,5-trimethyl-3-cyclohexenyl, 2,7,7-trimethyl-3-cycloheptenyl, 2,7,7-trimethyl-4-cycloheptenyl, 2,2,7-trimethyl-3-cycloheptenyl, 2,2,7-trimethyl-4-cycloheptenyl, 2,3,6-trimethyl-3-cyclohexenyl, 2,3,7-trimethyl-3-cycloheptenyl, 2,3,5-trimethyl-3-cyclopentenyl, 2,2,6,6-tetramethyl-3-cyclohexenyl, 2,2,5,5-tetramethyl-3-cyclopentenyl, 2,2,7,7-tetramethyl-3-cycloheptenyl, 2,3,5,5-tetramethyl-3-cyclopentenyl, 2,3,6,6-tetramethyl-3-cyclohexenyl, 2,3,7,7-tetramethyl-3-cycloheptenyl, 2,3,6,6-tetramethyl-3-cycloheptenyl, 2,3,5,5-tetramethyl-3-cyclohexenyl, 2,3,4,5-tetramethyl-3-cyclopentenyl, 2,3,4,5-tetramethyl-3-cyclohexenyl, etc.; bicyclic compounds, such as norbornyl, norcaranyl, norpinanyl, bicyclo [2.2.2] octyl, etc.; alkyl substituted bicyclic compounds, e.g., 6,6-dimethyl-bicyclo [3.1.1] heptyl, 6,7,7-trimethylnorbornyl (bornyl or camphanyl), pinanyl, thujanyl, caranyl, fenchyl, 2-norbornylmethyl, etc.; unsubstituted and alkyl-substituted bicycloalkenes such as norbornenyl, norpinenyl, norcarenyl, 2-(4-norbornenyl)methyl, pinenyl, carenyl, fenchenyl, etc.; and tricyclo compounds such as adamantyl and alkyl-substituted adamantyl, etc

The preferred $R_1$ is cycloalkyl or bicycloalkyl or alkyl-substituted cycloalkyl or bicycloalkyl, especially where the alkyl group is in the $\beta$ or $\beta'$ positions. Further, preference exists for compounds in which $R_1$ is a cycloalkyl with two, three or four alkyl groups in the $\beta$, $\beta'$ positions such as $\beta$, $\beta$, $\beta'$, $\beta'$-tetraalkyl-substituted cyclopentyl, cyclobutyl, cyclohexyl, and cycloheptyl, as well as $\beta$, $\beta$, $\beta'$-trialkyl substituted cyclobutyl, cyclopropyl, cyclohexyl, cyclopentyl, and cycloheptyl, and fenchyl. Also preferred are $\beta$-alkylcycloalkyls in which the alkyl group is isopropyl or tertiary butyl.

These novel compounds are effective sweetness agents when used alone or in combination with other sweeteners in an ingesta, e.g., foodstuffs or pharmaceuticals. For example, other natural and/or artificial sweeteners which may be used with the novel compounds of the present invention include sucrose, fructose, corn syrup solids, dextrose, xylitol, sorbitol, mannitol, acetosulfam, thaumatin, invert sugar, saccharin, thiophene saccharin, meta-aminobenzoic acid, meta-hydroxybenzoic acid, cyclamate, chlorosucrose, dihydrochalcone, hydrogenated glucose syrups, aspartame (L-aspartyl-L-phenylalanine methyl ester) and other dipeptides, glycyrrhizin and stevioside and the like. These sweeteners when employed with the sweetness agents of the present invention, it is believed, could produce synergistic sweetness responses.

Furthermore, when the sweetness agents of the present invention are added to ingesta, the sweetness agents may be added alone or with nontoxic carriers such as the abovementioned sweeteners or other food ingredients such as acidulants and natural and artificial gums. Typical foodstuffs, and pharmaceutical preparations, in which the sweetness agents of the present invention may be used are, for example, beverages including soft drinks, carbonated beverages, ready to mix beverages and the like, infused foods (e.g. vegetables or fruits), sauces, condiments, salad dressings, juices, syrups, desserts, including puddings, gelatin and frozen desserts, like ice creams, sherbets, icings and flavored frozen desserts on sticks, confections, toothpaste, mouthwash, chewing gum, cereals, baked goods, intermediate moisture foods (e.g. dog food) and the like.

In order to achieve the effects of the present invention, the compounds described herein are generally added to the food product at a level which is effective to perceive sweetness in the food stuff and suitably is in an amount in the range of from about 0.0005 to 2% by weight based on the consumed product. Greater amounts are operable but not practical. Preferred amounts are in the range of from about 0.001 to about 1% of the foodstuff. Generally, the sweetening effect provided by the present compounds are experienced over a wide pH range, e.g. 2 to 10 preferably 3 to 7 and in buffered and unbuffered formulations.

It is desired that when the sweetness agents of this invention are employed alone or in combination with another sweetner, the sweetener or combination of sweeteners provide a sucrose equivalent in the range of from about 2 weight percent to about 40 weight percent and more preferably from about 3 weight percent to about 15 weight percent in the foodstuff or pharmaceutical.

A taste procedure for determination of sweetness merely involves the determination of sucrose equivalency. Sucrose equivalence for sweeteners are readily determined. The amount of a sweetener that is equivalent to a given weight percent sucrose can be determined by having a panel of tasters taste solutions of a sweetener at known concentrations and match its sweetness to standard solutions of sucrose.

In order to prepare compounds of the present invention, several reaction schemes may be employed. In one reaction scheme, compounds of general formula II (protected $\alpha$-aminodicarboxylic acid) and III (amino-ester compound) are condensed to form compounds of general formula IV. Subsequent removal of protecting groups B and Z from compounds of general formula IV give the desired compounds of general formula I:

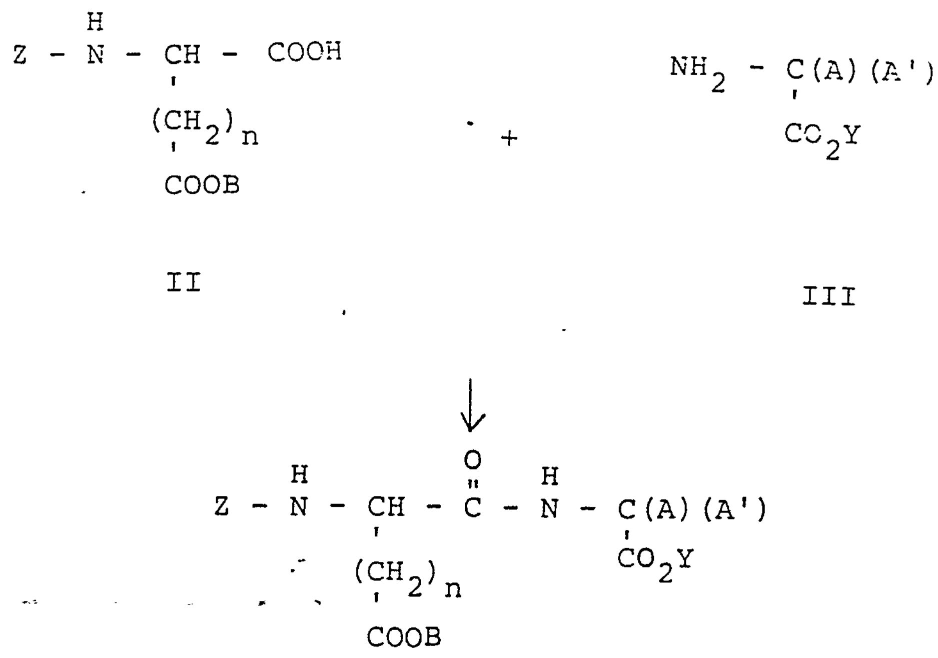

II

III

IV

In these, group Z is an amino protecting group, B is a carboxyl protecting group, and A, A', Y, and n have the same meaning as previously described. A variety of protecting groups known in the art may be employed. Examples of many of these possible groups may be found in "Protective Groups in Organic Synthesis" by T.W. Green, John Wiley and Sons, 1981. Among the preferred groups that may be employed are benzyloxycarbonyl for A and benzyl for B.

Coupling of compounds with general formula III to compounds having general formula IV employs established techniques in peptide chemistry. One such technique uses dicyclohexylcarbodiimide - (DCC) as the coupling agent. The DCC method may be employed with or without additives such as 4-dimethylaminopyridine or copper (II). The DCC coupling reaction generally proceeds at room temperature, however, it may be carried out from about -20° to 50°C. in a variety of solvents inert to the reactants. Thus suitable solvents include, but are not limited to, N,N-dimethyl-formamide, methylene chloride, toluene and the like. Preferably the reaction is carried out under an inert atmosphere such as argon or nitrogen. Coupling usually is complete within 2 hours but may take as long as 24 hours depending on reactants.

Various other methods can be employed to prepare the desired compounds. The following illustrates such methods using aspartic acid as the amino dicarboxylic acid.

For example, U.S. Patents 3,786,039; 3,833,553; 3,879,372 and 3,933,781 disclose the reaction of N-protected aspartic anhydrides with amino acids and amino acid derivatives to yield the desired products. These N-protected aspartic anhydrides can be reacted with compounds of formula III by methods disclosed in the above patents. As described in U.S. Patent 3,786,039 compounds of formula III can be reacted directly in inert organic solvents with L-aspartic anhydride having its amino group protected by a formyl, carbobenzloxy, or p-methoxycarbobenzloxy group which is subsequently removed after coupling to give compounds of general formula I. The N-acyl-L-aspartic anhydrides are prepared by reacting the corresponding acids

with acetic anhydride in amounts of 1.0 -1.2 moles per mole of the N-acyl-L-aspartic acid at 0° to 60°C in an inert solvent. The N-acyl-L-aspartic anhydrides are reacted with preferably 1 to 2 moles of compounds of formula III in an organic solvent capable of dissolving both and inert to the same. Suitable solvents are, but not limited to, ethyl acetate, methyl propionate, tetrahydrofuran, dioxane, ethyl ether, N,N-dimethylformamide and benzene. The reaction proceeds smoothly at 0° to 30°C. The N-acyl group is removed after coupling by catalytic hydrogenation with palladium on carbon or with HBr or HCl in a conventional manner. U.S. Patent No. 3,879,372 discloses that this coupling method can also be performed in an aqueous solvent at a temperature of -10° to 50°C and at a pH of 4-12.

Another method for the synthesis of the desired compounds is the reaction of compounds of formula III with suitable aspartic acid derivatives in which protecting groups have been attached to the amino and beta-carboxy groups and the alpha carboxy group has been converted to a reactive ester function. As disclosed in U.S. Patent 3,475,403 these coupled products may be deprotected as described to yield the desired compounds of formula I.

An alternative scheme to the desired coupled compounds involves reaction of compounds of formula III with L-aspartic acid N-thiocarboxyanhydride by the method of Vinick and Jung, Tet. Lett., 23, 1315-18 (1982). An additional coupling method is described by T. Miyazawa, Tet. Lett., 25, 771 (1984).

Compounds of general formula III are synthesized using art recognized techniques. For example, compounds of formula III can be synthesized by standard esterification methods known in the art by reacting the free acid or acid functional equivalents, such as esters or anhydrides, with the corresponding alcohols under ester-forming conditions, as for example in the presence of mineral acids, such as hydrochloric or sulfuric acids or organic acids, such as p-toluene-sulfonic acids. Reaction temperatures are in the range of -78° to reflux. The reaction is carried out in a solvent that will dissolve both reactants and is inert to both as well. Solvents include, but are not limited to methylene chloride, diethyl ether, tetrahydrofuran, dimethylsulfoxide, N,N-dimethylformamide, and the like.

With regard to the removal of protecting groups from compounds of formula IV and N-protected precursors of formula III, a number of deprotecting techniques are known in the art and can be utilized to advantage depending on the nature of the protecting groups. Among such techniques is catalytic hydrogenation utilizing palladium on carbon or transfer hydrogenation with 1,4-cyclohexadiene. Generally the reaction is carried at room temperature but may be conducted from 5 to 65°C. Usually the reaction is carried out in the presence of a suitable solvent which may include, but are not limited to water, methanol, ethanol, dioxane, tetrahydrofuran, acetic acid, t-butyl alcohol, isopropanol or mixtures thereof. The reaction is usually run at a positive hydrogen pressure of 50 psi but can be conducted over the range of 20 to 250 psi. Reactions are generally quantitative taking 1 to 24 hours for completion.

In any of the previous synthetic methods the desired products are preferably recovered from reaction mixtures by crystallization. Alternatively, normal or reverse-phase chromatography may be utilized as well as liquid/liquid extraction or other means.

The desired compounds of formula I are usually obtained in the free acid form; they may also be recovered as their physiologically acceptable salts, i.e., the corresponding amino salts such as hydrochloride, sulfate, hydrosulfate, nitrate, hydrobromide, hydroiodide, phosphate or hydrophosphate; or the alkali metal salts such as the sodium, potassium, lithium, or the alkaline earth metal salts such as calcium or magnesium, as well as aluminum, zinc and like salts.

Conversion of the free peptide derivatives of formula I into their physiologically acceptable salts is carried out by conventional means, as for example, bringing the compounds of formula I into contact with a mineral acid, an alkali metal hydroxide, an alkali metal oxide or carbonate or an alkaline earth metal hydroxide, oxide, carbonate or other complexed form.

These physiologically acceptable salts can also be utilized as sweetness agents usually having increased solubility and stability over their free forms.

It is known to those skilled in the art that the compounds of the present invention having asymmetric carbon atoms may exist in racemic or optically active forms. All of these forms are contemplated within the scope of the invention.

The compounds of the present invention have one asymmetric site, which is designated by an asterik (*) in the formula below, and one pseudoasymmetric sites which is designated by a double asterik (**):

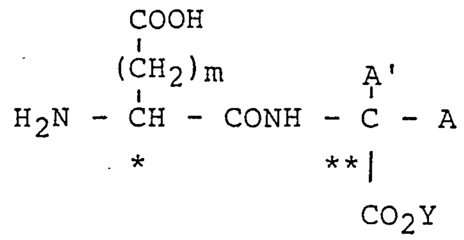

Whenever A is identical to A', the compounds of the present invention have only one asymmetric site, designated by the asterik, in the dicarboxylic acid moiety. Although both the D and L forms are possible; the preferred compounds are those in which the dicarboxylic acid group is in the L-configuration. Whenever, the groups A' and A are different, the carbon atom designated by the double asteriks becomes an asymmetric center and the compounds of the present invention will contain at least two asymmetric centers. Regardless, the configuration around each of the asymmetric sites, whenever present, may exist in either the D or L forms, and all possible stereoisomers are contemplated to be within the scope of the present invention. Since the dicarboxylic acid group is in the L-configuration, whenever an asymmetric center is present at the other carbon site, the compounds of the present invention are diastereomers, which can be separated, if desired, by art-recognized techniques, as, for example, chromatography. However, mixtures of at least any two stereoisomers exhibit sweetness properties and are useful as sweeteners.

The following examples further illustrate the invention.

EXAMPLE 1

L-Aspartyl-D-alanine(2,2,5,5-tetramethyl-1-cyclopentyl)ester

To a magnetically stirred solution of 10 g -(0.071 mol) 2,2,5,5-tetramethylcyclopentanone in 75 ml of dry tetrahydrofuran at 0°C under argon is added 2.69 g (0.071 mol) of lithium aluminum hydride. When the reduction was complete, ethyl acetate was introduced dropwise to destroy unreacted lithium aluminum hydride. 25 mls of water is then added, followed by 300 mls of diethyl ether. The organic phase is washed with 100 mls of water, and dried over $MgSO_4$. Filtration followed by evaporation afforded 8.23 g of 2,2,5,5-tetramethylcyclopentanol.

To a stirred solution of 9.59 g (0.043 mol) N-Cbz-alanine in 50 ml dry $CH_2Cl_2$ containing 8.96 g -(1 eq.) dicyclohexylcarbodiimide and 0.4 g dimethylaminopyridine (DMAP), all at 0°C, is added, via an addition funnel, 6.0 g (.043 mol) of 2,2,5,5-tetramethylcyclopentanol dissolved in 50 ml $CH_2Cl_2$. After stirring for 48 hours, the mixture is filtered, and the filtrate is washed with 5% HCl -(1x50ml), saturated $NaHCO_3$ (1x50 ml), and water -(1x50ml). The organic layer is separated, dried over $MgSO_4$ and evaporated to yield 10.11 g of 2,2,5,5-tetramethylcyclopentyl N-Cbz-D-alanine ester.

10 g of 2,2,5,5-Tetramethylcyclopentyl N-Cbz-D-alanine ester is dissolved in 100 ml $CH_3OH$ and hydrogenated over 5% Pd/C in a Paar hydrogenation apparatus. When the reaction is complete the mixture is filtered through Celite and concentrated to yield 2,2,5,5-tetramethylcyclopentyl alanine ester.

To a magnetically stirred solution of 2.14 g -(0.0099 mol) 2,2,5,5-tetramethylcyclopentyl alanine ester in 99 ml of dry dimethylformamide at 0°C under argon atmosphere is added 3.53 g N-Cbz-L-aspartic acid beta-benzyl ester followed by 1.33 g copper (II) chloride, and 2.06 g dicyclohexylcarbodiimide. This is stirred for 18 hours, after which the reaction mixture is poured into 200 ml 0.1 N HCl and extracted with 300 ml ethyl acetate. The organic phase is washed with saturated $NaHCO_3$ and then water, and dried over $MgSO_4$. Evaporated of the solvent yields 2.47 g N-(N'-Cbz-L-aspartyl beta-benzyl ester)-D-alanine 2,2,5,5-tetramethyl-1-cyclopentyl ester.

2.0 g N-(N'-Cbz-L-aspartyl beta-benzyl ester)-D-alanine 2,2,5,5-tetramethyl-1-cyclopentyl ester is dissolved in 50 ml $CH_3OH$ and hydrogenated over 5% Pd/C in a Parr apparatus. Upon completion of the reaction the mixture is filtered and concentrated to yield L-aspartyl-D-alanine 2,2,5,5-tetramethyl-1-cyclopentyl ester.$[\alpha]_D25$ (racemized) = -7.7°; $[\alpha]_D25$ (pure) = 21.9°.

Sweetness determination with this compound gave the following results:

| CONCENTRATION | SUCROSE EQUIVALENCE |
|---|---|
| 0.005 | 2.5 |
| 0.001 | 3.7 |
| 0.025 | 7.7 |
| .05 | 8.0 |

Similarly, by utilizing the appropriate alcohol, the following additional compounds are prepared:

N-L-Aspartylalanine(2,2,5,-trimethylcyclopentyl)-ester.

N-L-Aspartylalanine(2,5-dimethylcyclopentyl)ester.

N-L-Aspartylalanine(dicyclopropylmethyl)ester.

N-L-Aspartylalanine(fenchyl)ester.

N-L-Aspartylalanine(2-t-butylcyclopentyl)ester.

N-L-Aspartylalanine(1-t-butyl-1-cyclopropylmethyl)-ester.

N-L-Aspartylalanine(1-isopropyl-1-cyclopropylmethyl)ester.

EXAMPLE 2

N-(L-Aspartyl)-2-methylalanine(2,2,5,5-tetramethyl-1-cyclopentyl)ester

To a stirred solution of 2 g (.008 mol) of N-Cbz-2-aminoisobutyric acid in dry $CH_2Cl_2$ containing 1.9 g dicyclohexylcarbodiimide and 0.1 g dimethylaminopyridine (DMAP), all at 0°C, is added, via an addition funnel, 1.3 g of 2,2,5,5-tetramethylcyclopentanol dissolved in $CH_2Cl_2$. After stirring for 48 hours, the mixture is filtered, and the filtrate is washed with 5% HCl (1x50ml), saturated $NaHCO_3$0. (1x50ml), and water (1x50ml). The organic layer is separated, dried over $MgSO_4$ and evaporated to yield N-Cbz-2-aminoisobutyric acid 2,2,5,5-tetramethylcyclopentyl ester.

N-Cbz-2-aminoisobutyric acid 2,2,5,5-tetramethylcyclopentyl ester is dissolved in $CH_3OH$ and hydrogenated over 5% Pd/C in a Paar hydrogenation apparatus. When the reaction is complete the mixture is filtered through Celite and concentrated to yield 2-aminoisobutyric acid 2,2,5,5-tetramethylcyclopentyl ester (600 mg).

To a magnetically stirred solution of 0.6 g 2-aminoisobutyric acid 2,2,5,5-tetramethylcyclopentyl ester in 20 ml of dry dimethylformamide at 0°C under argon atomosphere was added 1.02 g N-Cbz-L-aspartic acid beta-benzyl ester followed by 0.38 g of copper (II) chloride and 0.58 g dicyclohexylcarbodiimide. This is stirred for 18 hours, after which the reaction mixture is poured into 0.1 N HCl and extracted with ethyl acetate. The organic phase is washed with saturated $NaHCO_3$ and then water, and dried over $MgSO_4$. Evaporation of the solvent yields N-(N'-Cbz-L-Aspartyl beta-benzyl ester)-2-aminoisobutryic acid -(2,2,5,5-tetra-methyl-1-cyclopentyl)ester.

N-(N'-Cbz-L-Aspartyl beta-benzyl ester)-2-aminoisobutyric acid 2,2,5,5-tetra-methyl-1-cyclopentyl ester is dissolved in $CH_3OH$ and hydrogenated over 5% Pd/C in a Parr apparatus. Upon completion of the reaction the mixture is filtered and concentrated to yield crude N-(L-Aspartyl)-2-methylalanine (2,2,5,5-tetramethyl-1-cyclopentyl)ester. Purification of the final product was done by reverse-phase chromatography on a Whatman Magnum 20 ODS-3 $C_{18}$ column; solvent system:40% methanol in $H_2O$. NMR - ($CDCl_3$/DMSO) : $\delta$ 0.90 (s, 6H), 1.00 (s, 6H), 1.25-1.5 (m, 4H), 1.50 (s, 6H), 2.50-2.70 (q. of d., 2H), 3.50, 3.85 (br.s, 1H), 4.85 (br.s).

Sweetness determination with this compound gave the following results:

| CONCENTRATION | SUCROSE EQUIVALENCE |
|---|---|
| 0.005 | 2.00 |
| 0.010 | 3.25 |
| 0.025 | 5.75 |

Similarly, by utilizing the appropriate alcohol, the following additional compounds are prepared:

N-L-Aspartyl 2-methylalanine(2,2,5,-trimethylcyclopentyl)ester.

N-L-Aspartyl 2-methylalanine(2,5-dimethylcyclopentyl)ester.

N-L-Aspartyl 2-methylalanine(dicyclopropylmethyl)-ester.

N-L-Aspartyl 2-methylalanine(fenchyl)ester.

N-L-Aspartyl 2-methylalanine(2-t-butylcyclopentyl)-ester.

N-L-Aspartyl 2-methylalanine(1-t-butyl-1-cyclopropylmethyl)ester.

N-L-Aspartyl 2-methylalanine(1-isopropyl-1-cyclopropylmethyl)ester.

EXAMPLE 3

N-(L-Aspartyl)-1-amino-1-cyclopropanecarboxylic acid (2,2,5,5-tetramethyl-1-cyclopentyl)ester

To a stirred solution of N-Cbz-1-aminocyclopropane carboxylic acid in dry $CH_2Cl_2$ containing dicyclohexylcarbodiimide and dimethylaminopyridine (DMAP), all at 0°C, is added, via an addition funnel, 2,2,5,5-tetramethylcyclopentanol dissolved in $CH_2Cl_2$. After stirring for 48 hours, the mixture is filtered, and the filtrate is washed with 5% HCl (1x50ml), saturated $NaHCO_3$ - (1x50 ml), and water (1x50ml). The organic layer is separated, dried over $MgSO_4$ and evaporated to yield N-Cbz-1-aminocyclopropanecarboxylic acid 2,2,5,5-tetramethylcyclopentyl ester.

N-Cbz-1-aminocyclopropanecarboxylic acid 2,2,5,5-tetramethylcyclopentyl ester is dissolved in absolute alcohol at 0°C in an ultrasound bath. Palladium on carbon (10%). is added. The hydrogen source, 1,4-cyclohexadiene, is added, and ultrasound is commenced for eight minutes. The slurry is then filtered through a bed of Celite with ethyl alcohol. The solvent is removed by rotary evaporation to yield 1-aminocyclopropylcarboxylic acid 2,2,5,5-tetramethylcyclopentyl ester.

To a magnetically stirred solution of 1-aminocyclopropane carboxylic acid 2,2,5,5-tetramethylcyclopentyl ester in dry dimethylformamide at 0°C under argon atmosphere is added N-Cbz-L-aspartic acid beta-benzyl ester followed by copper (II) chloride and dicyclohexylcarbodiimide. This is stirred for 18 hours, after which the reaction mixture is poured into 0.1 N HCl and extracted with ethyl acetate. The organic phase is washed with saturated $NaHCO_3$ and then water, and dried over $MgSO_4$. Evaporation of the solvent yields N-(N'-Cbz-L-Aspartyl beta-benzyl ester)-1-amino-1-cyclopropanecarboxylic acid 2,2,5,5-tetramethyl-1-cyclopentyl ester.

The N-(N'-Cbz-L-Aspartyl-beta-benzyl ester)-1-amino-1-cyclopropanecarboxylic acid 2,2,5,5-tetramethyl-1-cyclopentyl ester is dissolved in absolute alcohol at 0°C in an ultrasound bath. Palladium on carbon (10%) is added. The hydrogen source, 1,4-cyclohexadiene, is added, and ultrasound is commenced for eight minutes. The slurry is then filtered through a bed of Celite with ethyl alcohol. The solvent is removed by rotary evaporation to afford the final product.

Similarly, by utilizing the appropriate starting materials the following additional compounds are prepared: N-L-aspartyl 1-aminocyclopropane-1-carboxylic acid(2,2,5,-trimethylcyclopentyl)ester.

N-L-aspartyl 1-aminocyclopropane-1-carboxylic acid(2,5-dimethylcyclopentyl)ester.

N-L-aspartyl 1-aminocyclopropane-1-carboxylic acid (dicyclopropylmethyl)ester.

N-L-aspartyl 1-aminocyclopropane-1-carboxylic acid (fenchyl)ester.

N-L-aspartyl 1-aminocyclopropane-1-carboxylic acid (2-t-butylcyclopentyl)ester.

N-L-aspartyl 1-aminocyclopropane-1-carboxylic acid (1-t-butyl-1-cyclopropylmethyl)ester.

N-L-aspartyl 1-aminocyclopropane-1-carboxylic acid (1-isopropyl-1-cyclopropylmethyl)ester.

The sweetness determination with Aspartyl-1-aminocyclopropyl-1-carboxylic acid, 2,5-dimethyl-1-cyclopentyl ester gave the following results:

| % Compond | Sucrose Equivalents | Sweetness relative to Sucrose (x Sucrose) |
|---|---|---|
| 0.005 | 1.0 | 200 |
| .01 | 2.2 | 217 |
| .025 | 3.3 | 133 |

EXAMPLE 4

N-L-Aspartyl-0-methyl serine-(2,2,5,-trimethylcyclopentyl) ester

To a solution of 5g N-Cbz-D-serine 2,2,5-trimethylcyclopentyl ester in 50ml dry $CH_2Cl_2$ is added 2 equivalents of $Ag_2O$ and 2 equivalents of methyl iodide. After stirring for 2 hours, the mixture is filtered and concentrated to yield the methyl ether of N-Cbz-D-serine 2,2,5-trimethylcyclopentyl ester. 3g of N-Cbz-D-serine methyl ether 2,2,5-trimethylcyclopentyl ester is hydrogenated over 0.5g 10% Pd/C in 100ml $CH_3OH$. Upon completion, the mixture is filtered and concentrated to yield 3-methoxy-D-alanine 2,2,5-trimethylcyclopentyl ester. To a magnetically stirred solution of 2g of 3-methoxy-D-alanine 2,2,5-trimethylcyclopentyl ester in 100ml DMF at 0°C is added 1 equivalent of N-Cbz-L-aspartic acid-$\beta$ -benzyl ester followed by addition of 1 equivalent each of Cu(II) chloride and dicyclohexylcarbodiimide. After 18 hours the mixture is poured into 200ml 0.1 N HCl and extracted with 300ml ethyl acetate. The organic phase is washed with saturated $NaHCO_3$, and $H_2O$, dried over $MgSO_4$, filtered and concentrated to an oil that is reconstituted in 50ml $CH_3OH$ and hydrogenated over 0.5g 5% Pd/C. Filtration followed by concentration yields L-aspartyl-D-serine 2,2,5-trimethylcyclopentyl ester methyl ether.

Using the appropriate starting materials, the following dipeptides are additionally prepared:

N-L-Aspartyl-0-methyl-D-serine(2,5-dimethylcyclopentyl)ester.

N-L-Aspartyl-0-methyl-D-serine-(dicyclopropylmethyl)ester.

N-L-Aspartyl-0-methyl-D-serine(fenchyl)ester.

N-L -Aspartyl-0-methyl-D-serine(2-t-butylcyclopentyl)ester.

N-L-Aspartyl-0-methyl-D-serine(1-t-butyl-1-cyclopropylmethyl)ester.

N-L-Aspartyl-0-methyl-D-serine(1-isopropyl-1-cyclopropylmethyl)ester.

N-L-Aspartyl-0-methyl-D-serine-(2,2,5,5-tetramethylcyclopentyl)ester.

EXAMPLE 5

L-Aspartyl-serine-(2,2,5-trimethylcyclopentyl)ester

Into a suspension of N-Cbz-D-serine (5g) in 50ml of dry THF containing 1 equivalent of 2,2,5-trimethylcyclopentanol is bubbled dry hydrogen chloride gas at room temperature. Upon complete solution of the mixture, the reaction is refluxed for 5 hours, then concentrated. Ethyl acetate is added, and this is washed with saturated sodium bicarbonate, water, and dried over $MgSO_4$. Filtration followed by concentration yields N-Cbz-D-serine-2,2,5-trimethylcyclopentyl ester. 5g of this product is dissolved in 10ml methanol and hydrogenated in a Paar apparatus over 1g of 5% Pd/C to yield 2,2,5-trimethylcyclopentyl-D-serinate.

To a magnetically stirred solution of 0.1 mole 2,2,5-trimethylcyclopentyl-D-serinate in 100ml dry DMF at 0°C under an argon atmosphere is added 1 equivalent of N-Cbz-L-aspartic acid β-benzyl ester followed by addition of 1 equivalent each of Cu(II) chloride and dicyclohexylcarbodiimide. After 18 hours the mixture is poured into 200ml 0.1N HCl and extracted with 300ml ethyl acetate which is successively washed with saturated $NaHCO_3$, $H_2O$, and dried over $MgSO_4$. Filtration and evaporation yields N-Cbz-β-benzyl-L-aspartyl-D-serine 2,2,5-trimethylcyclopentyl ester. 2g N-Cbz-β-benzyl-L-aspartyl-D-serine 2,2,5-trimethylcyclopentyl ester in 50ml dry $CH_3OH$ is hydrogenated in a Paar apparatus over 5% Pd/C. Upon completion of the reaction, the mixture is filtered through Celite and concentrated to dryness to yield the final product.

Similarly, utilizing the appropriate starting materials the following additional compounds are prepared:

N-L-Aspartyl-D-serine(2,2,5-trimethylcyclopentyl)-ester.

N-L-Aspartyl-D-serine(2,5-dimethylcyclopentyl)-ester.

N-L-Aspartyl-D-serine(dicyclopropylmethyl)ester.

N-L-Aspartyl-D-serine(fenchyl)ester.

N-L-Aspartyl-D-serine(2-t-butylcyclopentyl)ester.

N-L-Aspartyl-D-serine(1-t-butyl-1-cyclopropylmethyl)ester.

N-L-Aspartyl-D-serine(1-isopropyl-1-cyclopropylmethyl)ester.

N-L -Aspartyl-D-serine-(2,2,5,5-tetramethylcyclopentyl)ester.

EXAMPLE 6

N-L-Aspartyl-D-alanine (1-methyl-1-cyclopentyl)-ester

A. N-carbobenzoxy-D-alanine (1-methyl-1-cyclopentyl)ester

To a magnetically stirred solution of 22.3 g (0.1 mol) N-Cbz-D-alanine in 50 mls of dry dichloromethane containing 0.5 mls of concentrated sulfuric acid at 0°C, was added dropwise a 10 g - (0.1 mol) sample of 1-methylcyclopentanol in 50 mls of dichloromethane. After 5 days of stirring at room temperature, the mixture was heated to reflux for 4 hours, after which the reaction was cooled to room temperature, washed with 100 mls of saturated $NaHCO_3$, 100 mls of water and dried over $MgSO_4$. Filtration followed by evaporation of the solvent yielded 1.81 g of the product.

B. D-Alanine(1-methylcyclopentyl)ester

1.8 g of the compound of part A was hydrogenated in 50 mls of methanol containing 0.5 g of 5% Pd/C catalyst in a Parr apparatus. The catalyst was filtered off, the solvent was removed by evaporation and 0.54 g of 1-methylcyclopentyl D-alanine ester was obtained.

C. Beta-benzyl-N-carbobenzoxy-L-aspartyl-D-alanine-(1-methyl-1-cyclopentyl) ester

To 0.54 g (0.0031 mol) of the product from B in 31 mls of dimethylformamide at 0°C under an argon atmosphere is added 1.11 g (0.0031 mol) of N-Cbz-L-aspartic acid, beta-benzyl ester, followed by 417 mg (1 equiv.) Cu (II) $Cl_2$ and 646 mg. (1 equiv.) dicyclohexylcarbodimide. This is stirred for 16 hours, after which it is poured into 200 mls of 0.1 N HCl and extracted with 3 x 100 ml of ethyl acetate. The organic phase was washed with 100 ml of water and dried over $MgSO_4$. Filtration and evaporation of the solvent yielded 2.30 g of beta-benzyl-N-carbobenzoxy L-aspartyl-D-alanine-(1-methyl-1-cyclopentyl) ester.

D.- N-L-aspartyl-D-alanine (1-methyl-1-cyclopentyl) ester

2.3 g of the product from C was hydrogenated over 0.5 g of Pd/C (5%) in methanol to yield L-aspartyl-D-alanine (1-methyl-1-cyclopentyl) ester.

Sweetness determination with this compound gave the following results:

| Percent of Compound | Sucrose Equivalence | Sweetness Value Relative to Sucrose (x Sucrose) |
|---|---|---|
| 0.05 | 20 | 40 |

EXAMPLE 7

N-L-Aspartyl-D-alanine(fenchyl)ester

A. N-carbobenoxy-D-alanine (fenchyl)ester

To a magnetically stirred solution of 7.36 g - (0.033 ml) of N-Cbz-D-alanine in 150 mls of dichloromethane at room temperature is added 1 equivalent of DCC and 0.75 g of DMAP. After cooling to 0°C, 5.08 g of fenchyl alcohol in 25 mls of dichloromethane is added dropwise. After 18 h, the urea is filtered off, and the filtrate is successively washed with 5% HCl (50 ml), saturated $NaHCO_3$ - (50 ml), water (50 ml) and dried over $MgSO_4$. Filtration followed by removal of the solvent yielded the product.

B. D-alanine fenchyl ester

3.09 g of N-Cbz-D-alanine fenchyl ester, as prepared in Example 7A above, was hydrogenated in 50 mls of methanol to yield 1.91 g of D-alanine fenchyl ester.

C. N-Cbz-beta-benzyl-L-aspartyl-D-alanine - (fenchyl) ester

Following the procedure of Example 6 C, N-Cbz-L-aspartic acid, beta benzyl ester, Cu (II)$Cl_2$ and dicyclohexylcarbodimide were added to a stirred solution of D-alanine fenchyl ester in 50 ml of dimethylformamide at 0°C under an argon atmosphere to produce the above product.

D. L-aspartyl-D-alanine-fenchyl ester

In the usual fashion, as described in Example 6D,N-Cbz-beta-benzyl-L-aspartyl-D-alanine fenchyl ester was hydrogenated in 50 mls of methanol to yield the above product.

EXAMPLE 8

N-L-Aspartylalanine(2,5-dimethylcyclopentyl)ester

A. N-carbobenzoxy-D-alanine (2,5-dimethylcyclopentyl)ester

To a magnetically stirred solution of 19.63 g - (0.088 mol) N-Cbz-D-alanine, 18.34 g (1 equiv.) of dicyclohexylcarbodiimide, and 0.88 g of 4-(dimethylamino)pyridine in 150 mls of dichloromethane at 0°C, is added 10 g (0.088 mol) of 2,5-dimethylcyclopentanol. After 48 hours, the reaction mixture is filtered to remove dicyclohexylurea and concentrated to a pale yellow oil, which is redissolved in ethyl acetate. This is successively washed with 100 mls of 5% HCl, 100 mls of saturated $NaHCO_3$, 100 mls of saturated NaCl and 100 mls of water, dried over $MgSO_4$ and filtered. Evaporation of the solvent afforded 23.7 g of N-Cbz-D-alanine, 2,5-dimethylcyclopentylester.

B. D-Alanine-(2,5-dimethylcyclopentyl) ester

5.08 g of the product from A was hydrogenated over 0.5 g of 5% Pd/C in 50 mls of $CH_3OH$ to yield 2.59 g of D-alanine-(2,5-dimethylcyclopentyl) ester.

C. N-Cbz-beta-benzyl-L-aspartyl-D-alanine(2,5-dimethylcyclopentyl) ester

To a solution of 9.45 g (0.05 mol) of the product from B in 300 mls of dry DMF at 0°C is added 17.85 g (1 eq.) of N-Cbz-beta-benzyl-L-aspartic acid, 6.72 g (1 eq.) copper (II) chloride and 10.42 g (1 eq.) dicyclohexylcarbodiimide. This, at 0°C under an argon atmosphere,is stirred for 18 hours. The mixture is filtered to remove the urea, and is poured onto 300 mls of 0.1 N HCl. The blue solution is extracted with 3 x 200 ml of diethyl ether and 3 x 200 ml of ethyl acetate. The combined organic phases were washed with 100 mls of $NaHCO_3$ with (saturated), 100 mls of saturated NaCl, and 100 mls of water, and dried over $MgSO_4$. Filtration and evaporation afforded 29.6 g of the above-identified product, and was purified by flash silica gel chromatography.

D. N-L-Aspartyl-D-alanine(2,5-dimethylcyclopentyl) ester

The product from C was hydrogenated in the usual fashion in methanol over 5% Pd/C to yield the final product.

Sweetness determination with this compound gave the following results:

| Concentration | Sucrose Equivalence | Sweetness relative to Sucrose (x Sucrose) |
|---|---|---|
| 0.005 | 1.16 | 233 |
| 0.01 | 2.3 | 230 |
| 0.025 | 4.82 | 193 |
| 0.050 | 7.50 | 150 |

EXAMPLE 9

N-L-Aspartyl-1-aminocyclopropane carboxylic acid (2,2,5,5-tetramethyl-1-cyclopentyl)ester

A. N-t-butoxycarbonyl-1-aminocyclopropanecarboxylic acid (1)

To a solution of 1-aminocyclopropanecarboxylic acid (3.03 g) in saturated aqueous sodium bicarbonate (150 ml) was added a solution of di-t-butyldicarbonate (9.82 g) in t-butanol (50 ml), and the resulting mixture was stirred overnight. Water was then added and the mixture was washed with ethyl acetate. The aqueous phase was separated,made acid to pH 1 with concentrated hydrochloric acid and extracted twice with ethyl acetate. The combined extracts were washed with saturated sodium chloride, dried over magnesium sulfate, and the solvent was evaporated to yield a white solid. - (4.77 g, 86%). NMR ($CDCl_3$) : $\delta$ 1.05-1.35 (m, 2H, cyclopropyl), 1.41 (s, 9H, t-butyl), 1.41-1.70 (m, 2H, cyclopropyl), 5.20, (br. s, 1H, NH), 9.25 (br. s, 1H, $CO_2H$).

B. 2,5-Dimethylcyclopentyl N-t-butoxycarbonyl-1-aminocyclopropanecarboxylate (2)

To a solution of 2,5-dimethylcyclopentanol - (0.55 g.), compound 1(0.97 g),and 4-(dimethylamino)pyridine (0.06 g.) in a methylene chloride (100 ml) was added dicyclohexylcarbodiimide (1.09 g.), and the resulting mixture was stirred overnight. The precipitated dicyclohexylurea was removed by filtration, and the filtrate was evaporated. Ethyl acetate was then added to the residue, and the mixture was filtered again. The filtrate was washed with 1 M hydrochloric acid, saturated aqueous sodium bicarbonate, and water, dried over magnesium sulfate, and the solvent was evaporated to a colorless oil (1.18 g. 83%). The product was purified by column chromotography on silica gel, 4:1 hexane:ethyl acetate, eluent.

NMR ($CDCl_3$) : $\delta$ 0.92 (t, 6H, $2CH_3$), 1.05-2.10 (m, 10H, cyclopentyl, cyclopropyl), 1.40 (s, 9H, t-Bu), 4.60 (dd., 1H, $CO_2C\underline{H}$), 5.05 (br. s, 1H, NH).

C. $\beta$-Benzyl-N-benzyloxycarbonyl-L-aspartyl-1-aminocyclopropanecarboxylic acid, 2,5-dimethylcyclopentyl ester (3)

A mixture of compound 2 (0.57 g), 95% ethanol (11 ml), water (7.5 mls) and concentrated hydrochloric acid (4 ml) was heated to reflux for 2 hours. The mixture was cooled, and 1 molar hydrochloric acid was added. The solution was washed with ethyl acetate. The separated aqueous phase was made basic with 1 M sodium hydroxide and extracted twice with ethyl acetate. The combined extracts were washed with saturated sodium chloride, dried over magnesium sulfate, and the solvent was evaporated to yield 0.22 g of a colorless oil. Dicyclohexylcarbodiimide (0.25 g) was added to a solution of the above oil, followed by N-benzyloxycarbonylaspartic acid, beta-benzyl ester - (0.40 g) and copper (II) chloride (0.17 g) in dimethyl formamide (10 mls). The resulting mixture was stirred overnight. The green mixture was then filtered to remove dicyclohexylurea, and 1 M hydrochloric acid was added to the filtrate. This was extracted twice with ethyl acetate, and the combined extracts were washed with 1 M hydrochloric acid, saturated sodium bicarbonate and saturated sodium chloride. The solution was dried over magnesium sulfate, and the solvent was evaporated to give 0.53 g of a yellow oil. This is purified by column chromotography on silica gel,4:1 hexane:ethyl acetate, eluent, to give the desired product as a white solid (0.33 g, 32%). NMR ($CDCl_3$) : $\delta$ 0.91 (t, 6H, $2CH_3$), 0.80-2.25 (m, 10H, cyclopropyl, cyclopentyl), 2.70 (dd. 1H, $J_1$=7Hz, $J_2$= 17Hz, aspartyl $CH_2$), 3.00 (dd., 1H, $J_1$=5Hz, $J_2$=17Hz, aspartyl $CH_2$), 4.30-4.70 (m, 2H, $CO_2C\underline{H}$, aspartyl $C\underline{H}$), 5.10 (s, 4H, $2PhC\underline{H}_2$), 5.87 (br. d, 1H, NH), 6.95 (br. s, 1H, NH), 7.30 (s, 10H, 2Ph).

D. L-Aspartyl 1-aminocyclopropanecarboxylic acid, 2,5-dimethylcyclopentyl ester

A mixture of compound 3 (0.31 g), 1,4-cyclohexadiene (0.46 g), 10% palladium-on-carbon (0.3 g) and 95% ethanol (10 ml) was placed in an ultrasonic bath for 10 minutes. The mixture was then filtered through Celite, and the solvent was evaporated to yield 130 mg of a colorless oil which solidified upon standing. This was purified by HPLC using a reverse phase $C_{18}$ column, 60% methanol in water as eluent to yield the desired product as a white solid (72 mg, 40%). MP:155.5-157°. FAB MS (m/z) : 313 (M+H, 22%) 217 (87%), 102 (69%), 88 (100%).

The compounds of this invention possess greater sweetness and higher stability in comparison to corresponding esters of the prior art.

**Claims**

1. A compound represented by the formula:

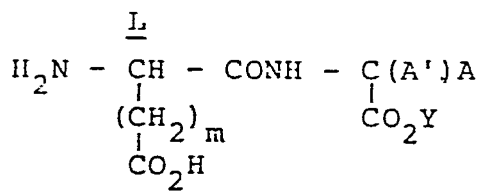

and food-acceptable salts thereof, wherein

A is hydrogen, alkyl containing 1-3 carbon atoms, hydroxyalkyl containing 1-3 carbon atoms or alkoxymethyl wherein the alkoxy contains 1-3 carbon atoms;

A' is hydrogen or alkyl containing 1-3 carbon atoms; alternatively

A and A' taken together with the carbon atom to which they are attached form cycloalkyl containing 3-4 carbon atoms;

Y is $-(CHR_2)_n-R_1$ or $-CHR_3R_4$;

$R_1$ is an alkyl-substituted cycloalkyl, cycloalkenyl bicycloalkyl or bicycloalkenyl wherein at least one alkyl is in the $\beta$-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

2. The compound of Claim 1 repesented by the formula:

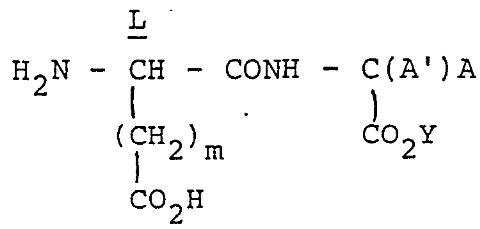

and food-acceptable salts thereof, wherein

A and A' are each hydrogen or alkyl containing 1-3 carbon atoms;

Y is $-(CHR_2)_n-R_1$ or $CHR_3R_4$;

$R_1$ is an alkyl-substituted cycloalkyl, cycloalkenyl bicycloalkyl or bicycloalkenyl wherein at least one alkyl is in the $\beta$-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

3. The compound of Claim 1 represented by the formula:

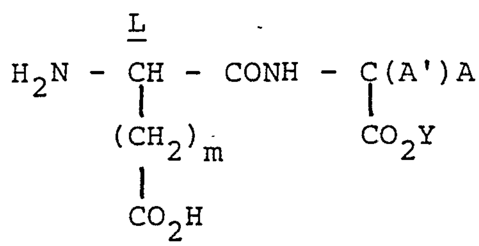

and food-acceptable salts thereof, wherein

A is hydroxyalkyl containing 1-3 carbon atoms;

A' is hydrogen or alkyl containing 1-3 carbon atoms;

Y is $-(CHR_2)_n-R_1$ or $-CHR_3R_4$;

$R_1$ is an alkyl-substituted cycloalkyl, cycloalkenyl, bicycloalkyl or bicycloalkenyl wherein at least one alkyl is in the $\beta$-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

4. The compound of Claim 1 represented by the formula:

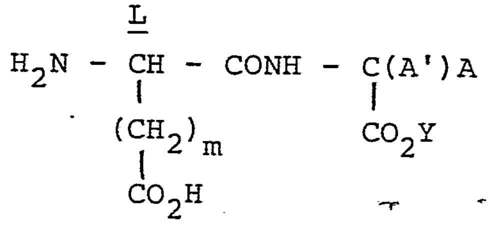

and food-acceptable salts thereof, wherein

A is alkoxymethyl wherein the alkoxy contains 1-3 carbon atoms;

A' is H or alkyl containing 1-3 carbon atoms;

Y is $-(CHR_2)_n-R_1$ or $-CHR_3R_4$;

$R_1$ is an alkyl-substituted cycloalkyl, cycloalkenyl bicycloalkyl or bicycloalkenyl wherein at least one alkyl is in the β-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

5. The compound of Claim 1 represented by the formula:

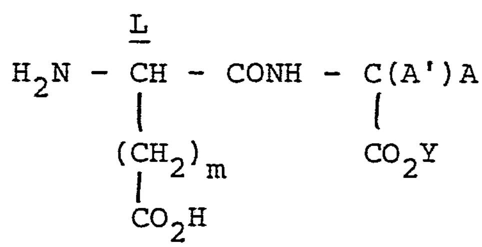

and food-accetable salts thereof, wherein

A and A' taken together with carbon atom to which they are attached form a cycloalkyl containing 3-4 carbon atoms;

Y is $-(CHR_2)_n-R_1$ or $-CHR_3R_4$;

$R_1$ is an alkyl-substituted cycloalkyl, cycloalkenyl bicycloalkyl or bicycloalkenyl wherein at least one alkyl is in the β-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

6. The compound of any of Claims 1-5 wherein n = 0.

7. The compound of any of Claims 1-6 wherein $R_1$ is mono-, di-, tri-or tetramethyl cycloalkyl or bicycloalkyl containing up to 10 carbon atoms.

8. The compound of any of Claims 1-6 wherein $R_1$ is an alkyl substituted cyclopentyl or cyclohexyl containing a total of up to 10 carbon atoms.

9. The compound of any of Claims 1-7 wherein $R_1$ is a β-methyl-substituted cycloalkyl or bicycloalkyl.

10. The compound of any of Claims 1-7 wherein $R_1$ is a β, β-or β, β'-dimethyl-substituted cycloalkyl or bicycloalkyl.

11. The compound of any of Claims 1-7 wherein $R_1$

is a β, β, β'-trimethyl-substituted cycloalkyl or bicycloalkyl.

12. The compound of any of Claims 1-7 wherein $R_1$ is a β, β, β', β'-tetramethyl substituted cycloalkyl or bicycloalkyl.

13. The compound of Claims 1-12 wherein $R_3$ and $R_4$ are cyclopropyl.

14. The compound of Claim 1 which is

N-L-Aspartylalanine(2,2,5,5-tetramethylcyclopentyl)-ester;

N-L-Aspartylalanine(2,2,5-trimethylcyclopentyl)-ester;

N-L-Aspartylalanine(2,5-dimethylcyclopentyl)ester;

N-L -Aspartylalanine(dicyclopropylmethyl)ester;

N-L-Aspartylalanine(fenchyl)ester;

N-L-Aspartylalanine(2-t-butylcyclopentyl)ester;

N-L-Aspartylalanine(1-t-butyl-1-cyclopropylmethyl)-ester;

N-L-Aspartylalanine(1-isopropyl-1-cyclopropylmethyl)ester;

N-L-Aspartyl 2-methylalanine(2,2,5-trimethylcyclopentyl)ester;

N-L -Aspartyl 2-methylalanine(2,5-dimethylcyclopentyl)ester;

N-L-Aspartyl 2-methylalanine(dicyclopropylmethyl)-ester;

N-L -Aspartyl 2-methylalanine(fenchyl)ester;

N-L-Aspartyl 2-methylalanine(2-t-butylcyclopentyl)-ester;

N-L-Aspartyl 2-methylalanine(1-t-butyl-1-cyclopropylmethyl)ester;

N-L-Aspartyl 2-methylalanine(1-isopropyl-1-cyclopropylmethyl)ester;

N-L-Aspartylserine(2,2,5,5-tetramethylcyclopentyl)-ester;

N-L-Aspartylserine(2,2,5-trimethylcyclopentyl)ester;

N-L-Aspartylserine(2,5-dimethylcyclopentyl)ester;

N-L-Aspartylserine(dicyclopropylmethyl)ester;

N-L-Aspartylserine(fenchyl)ester;

N-L-Aspartylserine(2-t-butylcyclopentyl)ester;

N-L-Aspartylserine(1-t-butyl-1-cyclopropylmethyl)-ester;

N-L-Aspartylserine(1-isopropyl-1-cyclopropylmethyl)ester;

N-L-Aspartyl 0-methylserine(2,2,5,5-tetramethylcyclopentyl)ester;

N-L-Aspartyl 0-methylserine(2,2,5-trimethylcyclopentyl)ester;

N-L-Aspartyl 0-methylserine(2,5-dimethylcyclopentyl)ester;

N-L-Aspartyl 0-methylserine(dicyclopropylmethyl)-ester;

N-L-Aspartyl 0-methylserine(fenchyl)ester;

N-L-Aspartyl 0-methylserine(2-t-butylcyclopentyl)-ester;

N-L-Aspartyl 0-methylserine(1-t-butyl-1-cyclopropylmethyl)ester;

N-L-Aspartyl 0-methylserine(1-isopropyl-1-cyclopropylmethyl)ester;

N-L-aspartyl 1-aminocyclopropane-1-carboxylic acid(2,2,5,5-tetramethylcyclopentyl)ester;

N-L-aspartyl 1-aminocyclopropane-1-carboxylic acid(2,2,5-trimethylcyclopentyl)ester;

N-L-aspartyl 1-aminocyclopropane-1-carboxylic acid(2,5-dimethylcyclopentyl)ester;

N-L-aspartyl 1-aminocyclopropane-1-carboxylic acid (dicyclopropylmethyl)ester;

N-L-aspartyl 1-aminocyclopropane-1-carboxylic acid(fenchyl) ester;

N-L-aspartyl 1-aminocyclopropane-1-carboxylic acid(2-t-butylcyclopentyl)ester;

N-L-aspartyl 1-aminocyclopropane-1-carboxylic

acid(1-t-butyl-1-cyclopropylmethyl)ester;

N-L-aspartyl 1-aminocyclopropane-1-carboxylic acid(1-isopropyl-1-cyclopropylmethyl)ester; or

N-L-aspartyl-D-alanine-(1-methyl-1-cyclopentyl)-ester.

15. An edible composition comprising a sweetening effective amount of a compound of any of claims 1-14.

CLAIMS FOR CONTRACTING STATE : AT

1. A process for the preparation of a compound of Formula I herein which comprises reacting a carboxylic acid or derivatives thereof of Formula II with an amine of Formula III under amide forming conditions and optionally removing the protecting groups when present and optionally forming pharmaceutically acceptable salts of said compounds.

2. The process of Claim 1 wherein the compound formed has the formula:

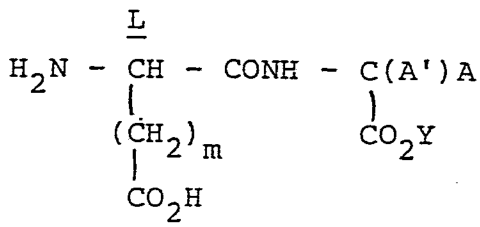

and food-acceptable salts thereof, wherein

A and A' are each hydrogen or alkyl containing 1-3 carbon atoms;

Y is $-(CHR_2)_n-R_1$ or $CHR_3R_4$;

$R_1$ is an alkyl-substituted cycloalkyl, cycloalkenyl bicycloalkyl or bicycloalkenyl wherein at least one alkyl is in the $\beta$-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

3. The process of Claim 1 wherein the compound formed has the formula:

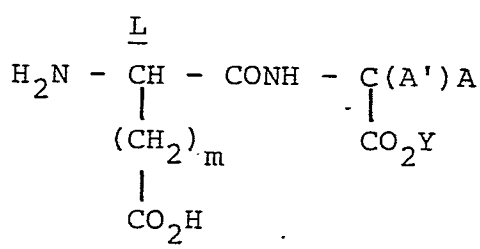

and food-acceptable salts thereof, wherein

A is hydroxyalkyl containing 1-3 carbon atoms;

A' is hydrogen or alkyl containing 1-3 carbon atoms;

Y is $-(CHR_2)_n-R_1$ or $-CHR_3R_4$;

$R_1$ is an alkyl-substituted cycloalkyl, cycloalkenyl, bicycloalkyl or bicycloalkenyl wherein at least one alkyl is in the $\beta$-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

4. The process of Claim 1 wherein the compound formed has the formula:

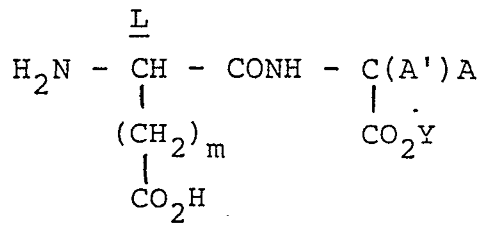

and food-acceptable salts thereof, wherein

A is alkoxymethyl wherein the alkoxy contains 1-3 carbon atoms;

A' is H or alkyl containing 1-3 carbon atoms;

Y is $-(CHR_2)_n-R_1$ or $-CHR_3R_4$;

$R_1$ is an alkyl-substituted cycloalkyl, cycloalkenyl bicycloalkyl or bicycloalkenyl wherein at least one alkyl is in the $\beta$-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

5. The process of Claim 1 wherein the compound formed has the formula:

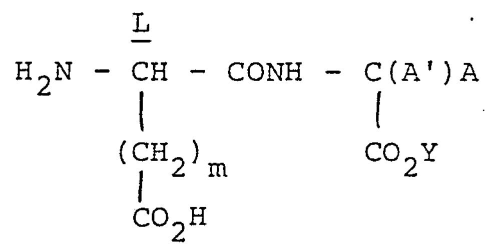

and food-accetable salts thereof, wherein

A and A' taken together with carbon atom to which they are attached form a cycloalkyl containing 3-4 carbon atoms;

Y is $-(CHR_2)_n-R_1$ or $-CHR_3R_4$;

$R_1$ is an alkyl-substituted cycloalkyl, cycloalkenyl bicycloalkyl or bicycloalkenyl wherein at least one alkyl is in the $\beta$-position of the cycloalkyl, cycloalkenyl, bicycloalkyl or bicycloalkenyl ring, containing up to 7 ring carbon atoms and a total of 12 carbon atoms;

$R_2$ is H or alkyl containing 1-4 carbon atoms;

$R_3$ and $R_4$ are each cycloalkyl containing 3-4 ring carbon atoms;

n = 0 or 1; and

m = 0 or 1.

6. The process of any of Claims 1-5 wherein n = 0.

7. The process of any of Claims 1-6 wherein $R_1$ is mono-, di-, tri-or tetramethyl cycloalkyl or bicycloalkyl containing up to 10 carbon atoms.

8. The process of any of Claims 1-6 wherein $R_1$ is an alkyl substituted cyclopentyl or cyclohexyl containing a total of up to 10 carbon atoms.

9. The process of any of Claims 1-7 wherein $R_1$ is a $\beta$-methyl-substituted cycloalkyl or bicycloalkyl.

10. The process of any of Claims 1-7 wherein $R_1$ is a $\beta$, $\beta$-or $\beta$, $\beta'$-dimethyl-substituted cycloalkyl or bicycloalkyl.

11. The process of any of Claims 1-7 wherein $R_1$ is a $\beta$, $\beta$, $\beta'$-trimethyl-substituted cycloalkyl or bicycloalkyl.

12. The process of any of Claims 1-7 wherein $R_1$ is a $\beta$, $\beta$, $\beta'$, $\beta'$-tetramethyl substituted cycloalkyl or bicycloalkyl.

13. The process of Claims 1-12 wherein $R_3$ and $R_4$ are cyclopropyl.

14. The process of Claim 1 wherein the compound formed is

N-L-Aspartylalanine(2,2,5,5-tetramethylcyclopentyl)-ester;

N-L-Aspartylalanine(2,2,5-trimethylcyclopentyl)-ester;

N-L -Aspartylalanine(2,5-dimethylcyclopentyl)ester;

N-L-Aspartylalanine(dicyclopropylmethyl)ester;

N-L -Aspartylalanine(fenchyl)ester;

N-L-Aspartylalanine(2-t-butylcyclopentyl)ester;

N-L-Aspartylalanine(1-t-butyl-1-cyclopropylmethyl)-ester;

N-L-Aspartylalanine(1-isopropyl-1-cyclopropylmethyl)ester;

N-L-Aspartyl 2-methylalanine(2,2,5-trimethylcyclopentyl)ester;

N-L -Aspartyl 2-methylalanine(2,5-dimethylcyclopentyl)ester;

N-L-Aspartyl 2-methylalanine(dicyclopropylmethyl)-ester;

N-L -Aspartyl 2-methylalanine(fenchyl)ester;

N-L-Aspartyl 2-methylalanine(2-t-butylcyclopentyl)-ester;

N-L-Aspartyl 2-methylalanine(1-t-butyl-1-cyclopropylmethyl)ester;

N-L-Aspartyl 2-methylalanine(1-isopropyl-1-cyclopropylmethyl)ester;

N-L-Aspartylserine(2,2,5,5-tetramethylcyclopentyl)-ester;

N-L-Aspartylserine(2,2,5-trimethylcyclopentyl)ester;

N-L-Aspartylserine(2,5-dimethylcyclopentyl)ester;

N-L-Aspartylserine(dicyclopropylmethyl)ester;

N-L-Aspartylserine(fenchyl)ester;

N-L-Aspartylserine(2-t-butylcyclopentyl)ester;

N-L-Aspartylserine(1-t-butyl-1-cyclopropylmethyl)-ester;

N-L-Aspartylserine(1-isopropyl-1-cyclopropylmethyl)ester;

N-L-Aspartyl 0-methylserine(2,2,5,5-tetramethylcyclopentyl)ester;

N-L-Aspartyl 0-methylserine(2,2,5-trimethylcyclopentyl)ester;

N-L-Aspartyl 0-methylserine(2,5-dimethylcyclopentyl)ester;

N-L-Aspartyl 0-methylserine(dicyclopropylmethyl)-ester;

N-L-Aspartyl 0-methylserine(fenchyl)ester;

N-L-Aspartyl 0-methylserine(2-t-butylcyclopentyl)-ester;

N-L-Aspartyl 0-methylserine(1-t-butyl-1-cyclopropylmethyl)ester;

N-L-Aspartyl 0-methylserine(1-isopropyl-1-cyclopropylmethyl)ester;

N-L-aspartyl 1-aminocyclopropane-1-carboxylic acid(2,2,5,5-tetramethylcyclopentyl)ester;

N-L-aspartyl 1-aminocyclopropane-1-carboxylic acid(2,2,5-trimethylcyclopentyl)ester;

N-L-aspartyl 1-aminocyclopropane-1-carboxylic acid(2,5-dimethylcyclopentyl)ester;

N-L-aspartyl 1-aminocyclopropane-1-carboxylic acid (dicyclopropylmethyl)ester;

N-L-aspartyl 1-aminocyclopropane-1-carboxylic acid(fenchyl)ester;

N-L-aspartyl 1-aminocyclopropane-1-carboxylic acid(2-t-butylcyclopentyl)ester;

N-L-aspartyl 1-aminocyclopropane-1-carboxylic acid(1-t-butyl-1-cyclopropylmethyl)ester;

N-L-aspartyl 1-aminocyclopropane-1-carboxylic acid(1-isopropyl-1-cyclopropylmethyl)ester; or

N-L-aspartyl-D-alanine-(1-methyl-1-cyclopentyl)-ester.

15. Use of a compound produced in accordance with the process of any of Claims 1-14 in a sweetening effective amount for the preparation of an edible composition.